(19) Europäisches Patentamt
European Patent Office
Office européen des brevets

(11) Veröffentlichungsnummer: **0 087 654**
**B1**

(12) **EUROPÄISCHE PATENTSCHRIFT**

(45) Veröffentlichungstag der Patentschrift: **30.04.86**

(51) Int. Cl.⁴: **C 07 D 209/28**

(21) Anmeldenummer: **83101351.1**

(22) Anmeldetag: **12.02.83**

(54) **Verfahren zur Herstellung von 1-(4-Chlorbenzoyl)-5-methoxy-2-methyl-3-indolacetoxyessigsäure.**

(30) Priorität: **26.02.82 DE 3206889**

(43) Veröffentlichungstag der Anmeldung: **07.09.83 Patentblatt 83/36**

(45) Bekanntmachung des Hinweises auf die Patenterteilung: **30.04.86 Patentblatt 86/18**

(84) Benannte Vertragsstaaten: **AT BE CH DE FR GB IT LI LU NL SE**

(56) Entgegenhaltungen:
**DE - A - 2 740 854**

**CHEMICAL ABSTRACTS, Band 90, Nr. 10, 5. März 1979, Seite 60, Nr. 87267x, Columbus, Ohio, USA**

**Die Akte enthält technische Angaben, die nach dem Eingang der Anmeldung eingereicht wurden und die nicht in dieser Patentschrift enthalten sind.**

(73) Patentinhaber: **Troponwerke GmbH & Co. KG, Berliner Strasse 156, D-5000 Köln 80 (DE)**

(72) Erfinder: **Boltze, Karl-Heinz, Dr., Ackerstrasse 8, D-5231 Borod (DE)**

(74) Vertreter: **Jesse, Ralf-Rüdiger, Dr. et al, Bayer AG Konzernverwaltung RP Patentabteilung, D-5090 Leverkusen 1 Bayerwerk (DE)**

**EP 0 087 654 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft ein neues chemisch eigenartiges und vorteilhaftes Verfahren zur Herstellung der bekannten 1-(4-Chlorbenzoyl)-5-methoxy-2-methyl-3-indolacetoxyessigsäure (Acemetacin entsprechend Formel I).

Für die Herstellung dieser bekannten Verbindung sind bereits eine Reihe von Verfahren bekannt geworden, vgl. z.B. DE-OS 2 234 651, DE-OS 2 257 867 und DE-OS 2 943 125.

Bei den bekannten Verfahren wurde die Carboxygruppe zunächst durch einen Benzylrest geschützt, so daß in einem letzten Reaktionsschritt eine katalytische Hydrierung des Benzylesters gemäß folgendem Reaktionsschema durchgeführt werden mußte.

**Reaktionsschema**

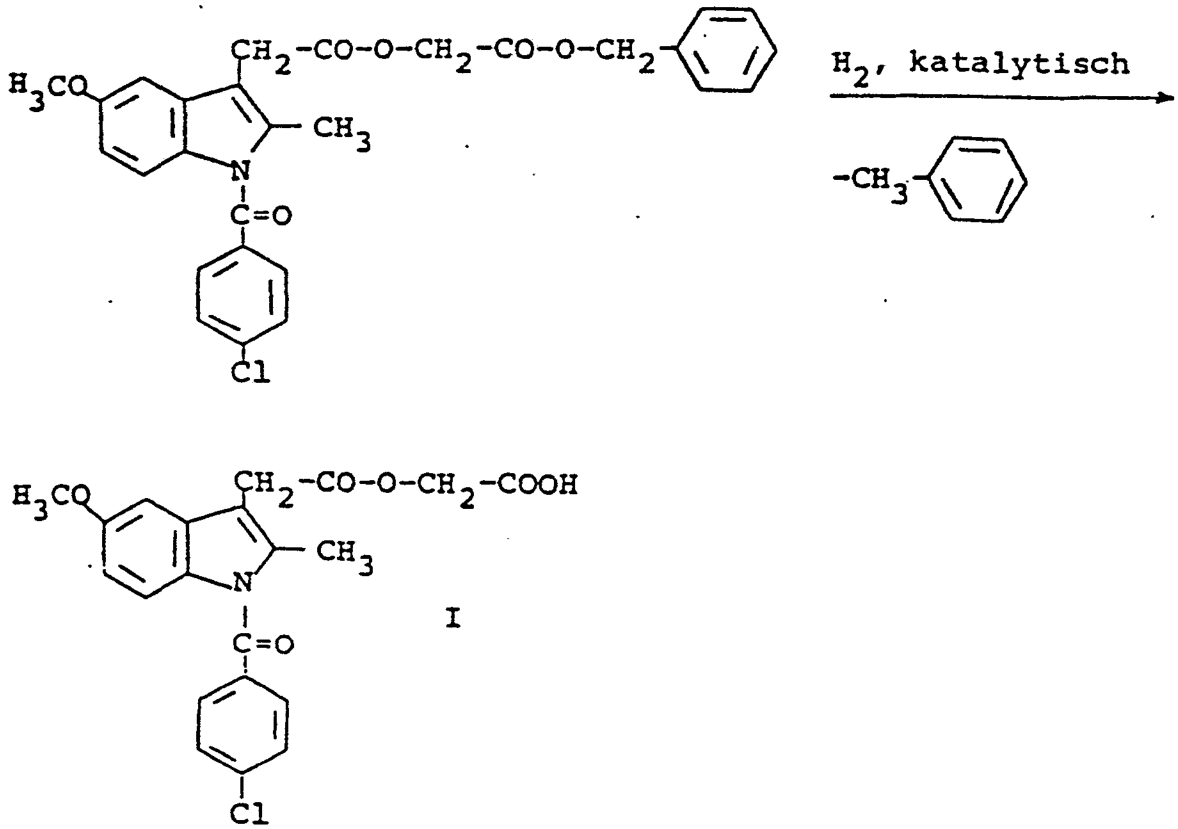


I

Bei dieser Abspaltung des Benzylrestes entsteht als Nebenprodukt stets die 1-Benzoyl-5-methoxy-2-methyl-3-indol acetoxyessigsäure, nachstehend Des-Chlor-Verbindung genannt. Diese unerwünschte Verunreinigung, die durch Abspaltung des Chlors aus dem Benzolring des 4-Chlorbenzoylrestes bis zu einer Menge von 0,5 % entsteht, muß in aufwendigen Reinigungsschritten anschlissend entfernt werden, was mit Ausbeuteverlusten verbunden ist.

Aus CA 90, 87627x ist die Umsetzung von Indometacin mit Thionylchlorid in Ethylether/Pyridin zu dem entsprechenden Säurechlorid bekannt, das mit Glykolsäure Acemetacin ergibt.

Es wurde ein Verfahren zur Herstellung von 1-(4-Chlor-benzoyl)-5-methoxy-2-methyl-3-indolacetoxyessigsäure gefunden, das dadurch gekennzeichnet ist, daß man das Derivat der Indolcarbonsäure

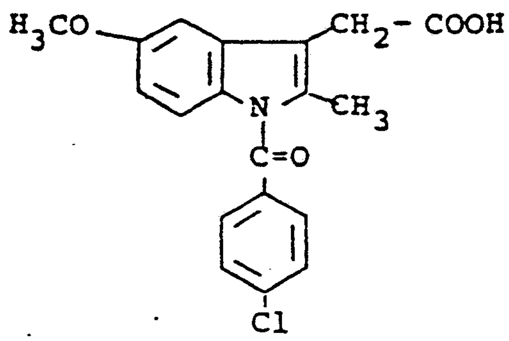

mit Verbindungen der allgemeinen Formel

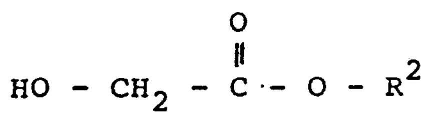

in welcher
$R^2$ für Wasserstoff oder Ammonium steht
in Gegenwart von inerten organischen Lösungsmitteln in einem Temperaturbereich von −10°C bis 80°C umsetzt.

Nach dem erfindungsgemänen Verfahren hergestelltes Acemetacin ist überraschenderweise frei von Deschlorverbindungen.

Das erfindungsgemäße Verfahren kann in Gegenwart von inerten organischen Lösungsmitteln wie Ether, z.B. Diethylether, Diisopropylether, Dioxan, Tetrahydrofuran, 1,2-Dimethoxyethan, chlorierte Kohlenwasserstoffe, z.B. Methylenchlorid, Chloroform, Dichlorethan, substituierte Amide, z.B. Dimethylformamid, N-Methylpyrrolidon, Aromaten, z.B. Toluol, Xylol, Ketone, z.B. Aceton, Methylethylketon (Butanon-2-) durchgeführt werden.

Die Umsetzung erfolgt in einem Temperaturbereich von −10°C bis 80°C, bevorzugt bei −10°C bis 50°C, besonders bevorzugt bei −5°C bis 20°C.

Verwendet man als Vertreter der allgemeinen Formel II die Hydroxyl-Indolcarbonsäurederivate, und Verbindungen der allgemeinen Formel III als Ausgangsstoffe, so kann der Reaktionsablauf durch das folgende Formelschema wiedergegeben werden:

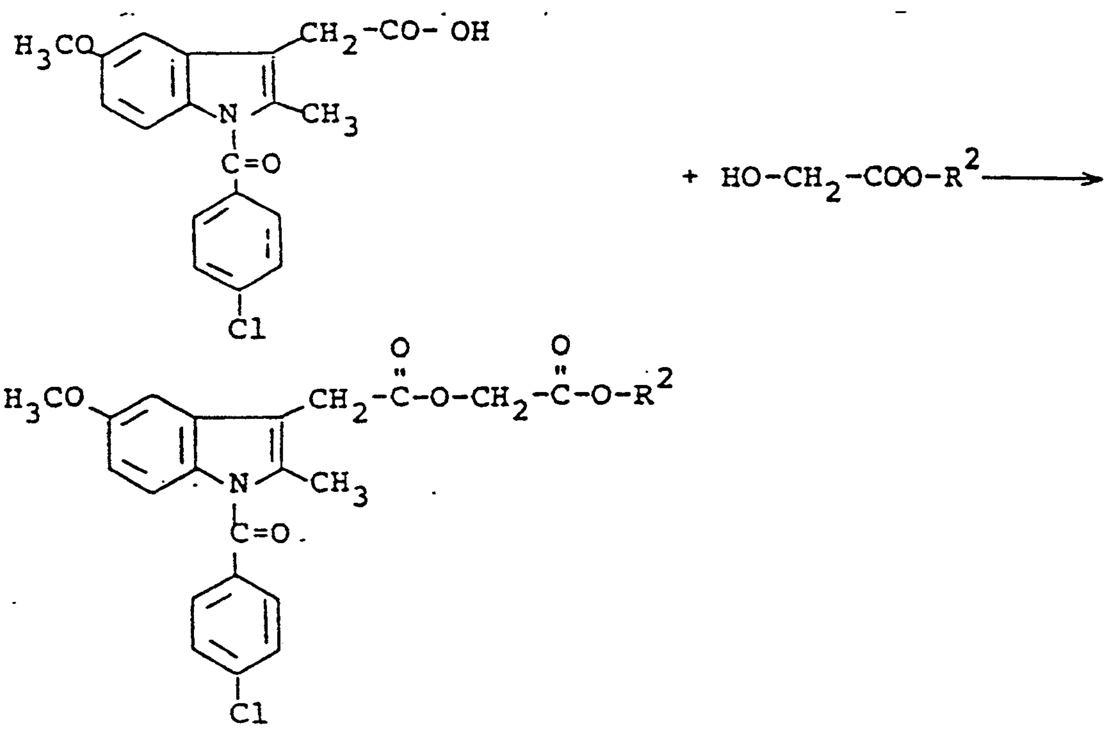

$R^2$ steht vorzugsweise für ein Ammoniumkation.

Die entstandenen Ammoniumverbindungen werden anschließend. durch Behandlung mit Säuren, in einfacher Weise in das Endprodukt I überführt.

Es war überraschend, daß nach diesen Verfahren die 1-(4-Chlorbenzoyl)-5-methoxy-2-methyl-3-indolacetoxyessigsäure in so reiner Form, d.h. frei von der störenden Des-Chlor-Verbindung, und in Ausbeuten von 60-70 % der Theorie entsteht.

Die als Ausgangsstoffe verwendeten Verbindungen der Formel II und III sind bekannt oder werden nach bekannten Verfahren hergestellt.

Die nach den erfindungsgemäßen Verfahren hergestellte Endverbindung I ist ein wertvoller pharmazeutischer Wirkstoff mit antiphlogistischer Wirkung, vgl. z.B. DE-PS 2 234 651.

Bei dem im vorliegenden Verfahren erfolgenden direkten Veresterung der Indolcarbonsäure und der Glykolsäure (III, $R^2$ H) gelangt man auf zwei Wegen zu 1-(4-Chlorben-zoyl)-5-methoxy-2-methyl-3-indolacetoxy-essigsäure (I) (vgl. Reaktionsschemata).

a) Reaktionsschema: Synthese von I durch direkte Veresterung in Gegenwart von H⊕

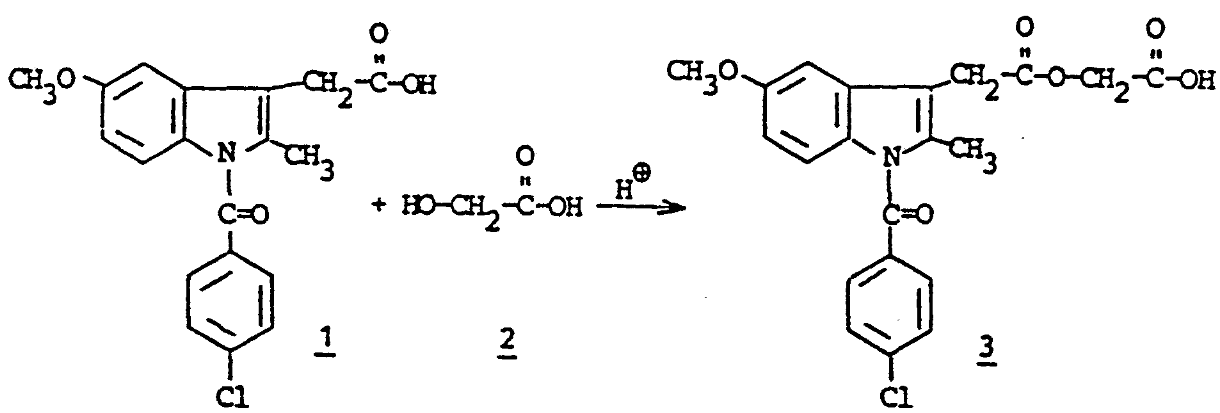


1 2 3

b) Reaktionsschema: Synthese von I durch direkte Veresterung in Gegenwart von DCC

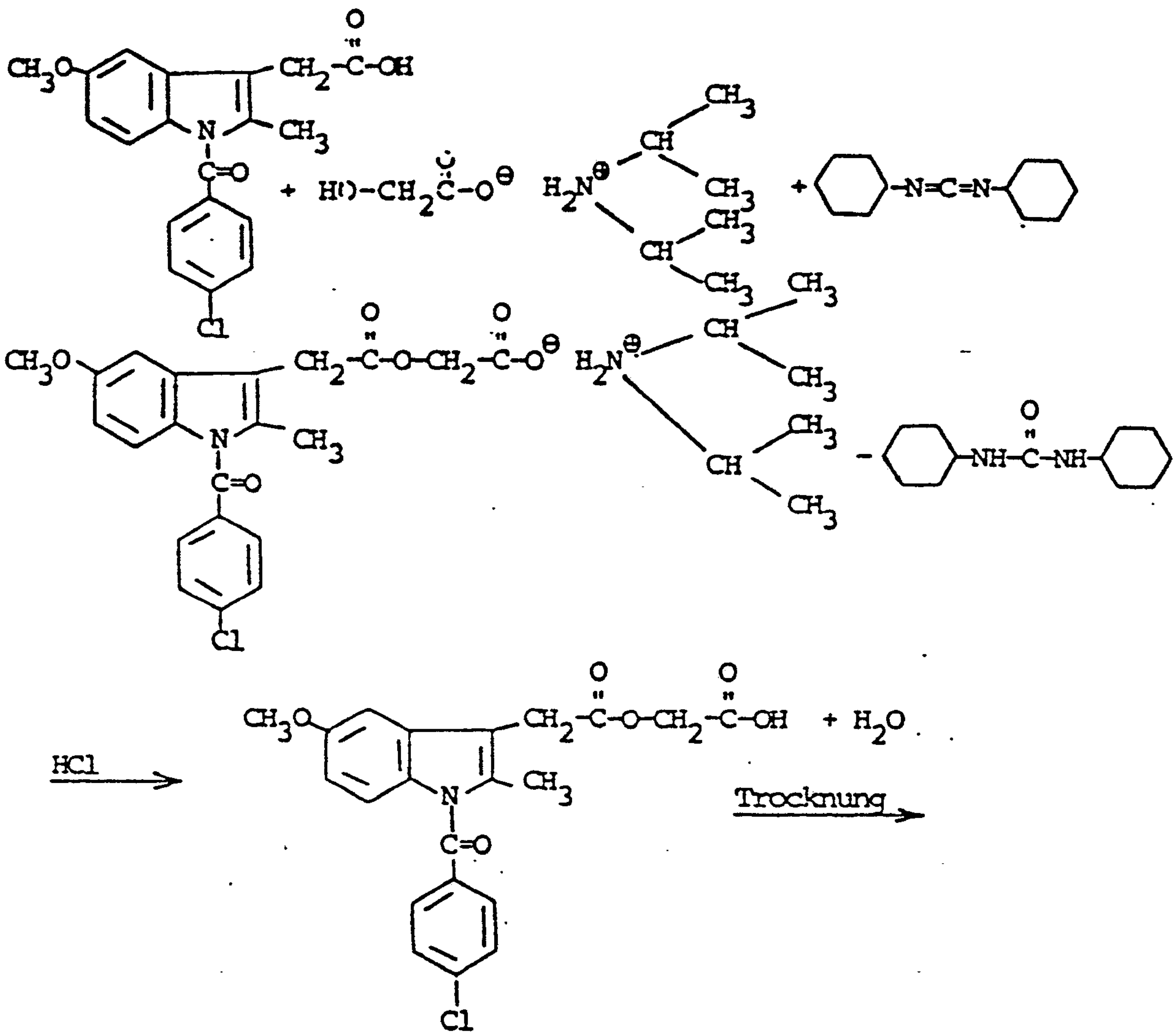


Einmal wird die Indolcarbonsäure (II) und die Glykolsäure (III, $R^2$ H) in einem inerten, mit Wasser nicht mischbaren Lösungsmittel, bevorzugt Toluol, mehrere Stunden am Wasserabscheider erhitzt. Bei dieser Reaktion kann sowohl die eigene Protonenaktivität der Glykolsäure als Katalysator dienen als auch die einer zugesetzten starken Säure, wie z.B. p-Toluolsulfonsäure.

Nach chromatographischer Reinigung erhält man I in reiner Form. Alternativ hierzu wird die direkte Veresterung durchgeführt, indem die Indolcarbonsgure (II) mit dem Diisopropylammoniumglykolat unter Feuchtigkeitsabschluß in Gegenwart eines Kondensationsmittels, wie z.B. Dicyclohexylcarbodiimid (DCC), zur Umsetzung gebracht wird. Man erhält das Diisopropylammoniumsalz I als farblose kristalline Substanz.

Als Lösungsmittel werden chlorierte Kohlenwasserstoffe, wie Chloroform, Methylenchlorid, letzteres bevorzugt, eingesetzt. Die Reaktionstemperaturen liegen zwischen 0°C und 50°C, bevorzugt bei 20°C.

Das gereinigte Ammoniumsalz von I überführt man durch Zusatz von wäßriger HCl in die freie Säure und kristallisiert sorgfältig und vollständig das farblose I-Monohydrat. Als Lösungsmittel werden Ketone oder Ether verwendet, die mit Wasser mischbar sind, bevorzugt Aceton oder Dioxan. Die Reaktionstemperaturen liegen zwischen 0°C und 60°C, bevorzugt 20°C oder 40°C.

Das hochreine Monohydrat verliert in einem schonenden Trocknungsprozeß seinen Wassergehalt und liefert I als gelbliche Kristalle, die bei 151° bis 152° schmelzen.

**Beispiel 1**

a) direkte Veresterung in Gegenwart von $H^+$

3,6 g (0,01 Mol) II (R OH) und 3,8 g (0,05 Mol) Glykolsäure werden in 40 ml Toluol suspendiert und unter Rühren am Wasserabscheider 64 h zum Sieden erhitzt. Wasserabscheidung: 0,8 ml.

Toluol destilliert man ab, mörsert den Rückstand und extrahiert mit $CH_2Cl_2$.

Der vom Lösungsmittel befreite ölige Rückstand wird an Kieselgel chromatografiert. (Fließmittel: Cyclohexan/Ethylacetat/Essigsäure: 10/10/1 v/v).

**Ausbeute:** 1,1 g 1-(4-Chlorbenzoyl)-5-methoxy-2-methyl-3-indolacetoxyessigsäure I = 26,4 % d.Th.

b) direkte Veresterung in Gegenwart von DCC

10,8 g II ($R^1$ OH) und 7,1 g Diisopropylammoniumglykolat (0,04 Mol) werden unter Rühren und Feuchtigkeitsabschluß bei Raumtemperatur in 60 ml $CH_2Cl_2$ gelöst und anschließend mit 6,2 g Dicyclohexylcarbodiimid (DCC) (0,03 Mol) versetzt. Man läßt 3 h bei Raumtemperatur nachreagieren und filtriert den entstandenen Niederschlag ab. Das Filtrat wird im Rotationsverdampfer unter Wasserstrahlvakuum vom Lösungsmittel befreit und dersirupöse Rückstand mit 160 ml Ether versetzt. Die Lösung läßt man bei Raumtemperatur über ein Wochenende stehen und filtriert das farblose kristalline Ammoniumsalz von I ab.

Die Überführung in I (freie Säure) erfolgt, indem unter Rühren das so gewonnene Ammoniumsalz in 11 ml Aceton und 5 ml $H_2O$ gelöst wird. Anschließend gibt man 8 ml,l n HCl zu dieser Lösung und impft an. Man rührt 1 h nach, bis die Kristallisation vollständig ist. Die farblose kristalline Substanz wird abgesaugt, mit $H_2O$ gut gewaschen und im Exsikkator bei 40°C im Wasserstrahlvakuum getrocknet.

**Ausbeute:** 3,35 g 1-(4-Chlorbenzoyl)-5-methoxy-2-methyl-3-indolacetoxyessigsäure-Monohydrat = 25,7 % d. Th.

Bei der Trocknung im Wasserstrahlvakuum bei 90°C bis zum vollständigen Wasserentzug entsteht I in gelblichen Kristallen, die bei 151 bis 152°C schmelzen.

**Patentansprüche:**

1. Verfahren zur Herstellung von 1-(4-Chlorbenzoyl)-5-methoxy-2-methyl-5-indolacetoxyessigsäure dadurch gekennzeichnet, daß man das Derivat der Indolcarbonsäure der Formel

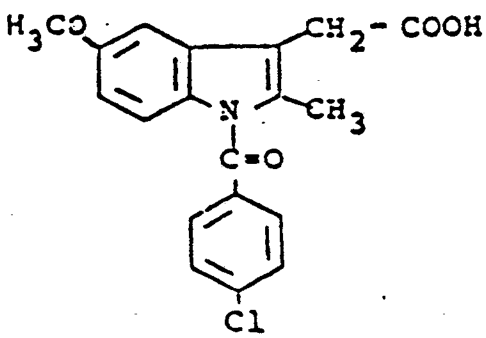

mit Verbindungen der allgemeinen Formel

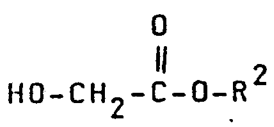

in welcher
$R^2$ für Wasserstoff oder Ammonium steht
in Gegenwart von inerten organischen Lösungsmitteln in einem Temperaturbereich von -10°C bis 80°C umsetzt.

2. Verfahren nach Anspruch 1, dadurch gekennzeichnet, daß die Umsetzung in einem Temperaturbereich von —10°C bis 50°C vorgenommen wird.

3. Verfahren nach den Ansprüchen 1 und 2, dadurch gekennzeichnet, daß es in Gegenwart einer Säure durchgeführt wird.

4. Verfahren nach den Ansprüchen 1 und 2, dadurch gekennzeichnet, daß es in Gegenwart eines Kondensationsmittels durchgeführt wird.

5. Verfahren nach den Ansprüchen 1 bis 4, dadurch gekennzeichnet, daß als inerte organische Lösungsmittel Ether, chlorierte Kohlenwasserstoffe, substituierte Amide, Aromaten und/oder Ketone eingesetzt werden können.

6. Verfahren nach den Ansprüchen 1 bis 5, dadurch gekennzeichnet, daß das Ammoniumsalz der Glykolcarbonsäure eingesetzt wird.

**Claims**

Process for the preparation of 1-(4-chlorobenzoyl)-5-methoxy-2-methyl-3-indoleacetoxyacetic acid, characterised in that the derivative of the indolecarboxylic acid of the formula

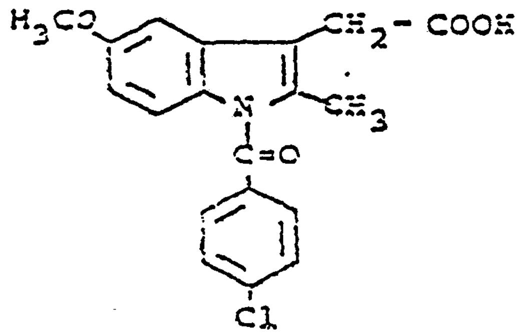

is reacted with compounds of the general formula

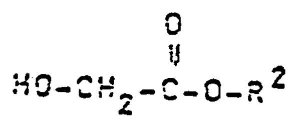

in which

$R^2$ represents hydrogen or ammonium, in the presence of inert organic solvents in a temperature range of −10°C to 80°C.

2. Process according to Claim 1, characterised in that the reaction is carried out in a temperature range of −10°C to 50°C.

3. Process according to Claims 1 and 2, characterised in that it is carried out in the presence of an acid.

4. Process according to Claims 1 and 2, characterised in that it is carried out in the presence of a condensing agent.

5. Process according to Claims 1 to 4, characterised in that ethers, chlorinated hydrocarbons, substituted amides, aromatics and/or ketones can be used as the inert organic solvents.

6. Process according to Claims 1 to 5, characterised in that the ammonium salt of glycolcarboxylic acid is used.

**Revendications**

1. Procédé de préparation d'acide 1-(4-chlorobenzoyl)-5-méthoxy-2-méthyl-3-indolacétoxy-acétique, caractérisé en ce qu'on fait réagir le dérivé de l'acide indole-carboxylique de formule:

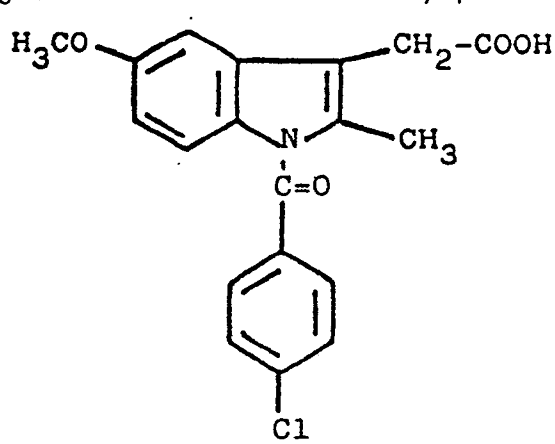

avec des composés de formule générale:

$$HO-CH_2-\overset{O}{\overset{\|}{C}}-O-R^2$$

dans laquelle

$R^2$ représente l'hydrogène ou l'ammonium, en présence de solvants organiques inertes et à une température se situant dans l'intervalle allant de -10°C à 80°C.

2. Procédé selon la revendication 1, caractérisé en ce qu'on effectue la réaction à une température se situant dans l'intervalle allant de -10°C à 50°C.

3. Procédé selon les revendications 1 et 2, caractérisé en ce qu'on l'effectue en présence d'un acide.

4. Procédé selon les revendications 1 et 2, caractérisé en ce qu'on l'effectue en présence d'un agent de condensation.

5. Procédé selon les revendications 1 à 4, caractérisé en ce que, comme solvants organiques inertes, on peut utilisier des éthers, des hydrocar bures chlorés, des amides substitués, des hydrocarbures aromatiques et/ou des cétones.

6. Procédé selon les revendications 1 à 5, caractérisé en ce qu'on utilise le sel d'ammonium de l'acide glycolcarboxylique.